# DEMANDE DE BREVET EUROPEEN

(11) **EP 0 695 747 A1**
(43) Date de publication de la demande: **07.02.1996**
(21) Numéro de dépôt: 95401626.7
(22) Date de dépôt: 06.07.1995
(51) Int. Cl.: C07D 401/04, A61K 31/47

(54) **Dérivés N-oxydes de 1-(7-chloroquinoléin-4-yl)pyrazole-3-carboxamides substitués, procédé pour leur préparation et les compositions pharmaceutiques les contenant**

(30) Priorité: 08.07.1994 FR 9408459
(71) Demandeur: SANOFI, F-75008 Paris (FR)
(72) Inventeur: Boigegrain, Robert, F-34820 Assas (FR); Jeanjean, Francis, F-34270 Valflaunes (FR); Gully, Danièle, F-31600 Muret (FR); Pradines, Antoine, F-31120 Roquettes (FR)
(74) Mandataire: Gillard, Marie-Louise

(57) **Abrégé**

La présente invention a pour objet des composés de formule : 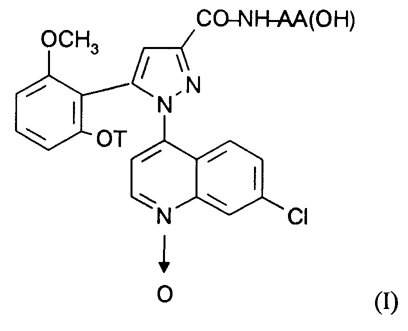
dans laquelle
- T représente l'hydrogène, un alkyle en C₁-C₄, un cycloalkyle en C₃-C₈, un (C₃-C₈) cycloalkylméthyle, un méthoxyéthyle;
- le groupe -NH-AA(OH) représente le résidu d'un acide aminé de formule 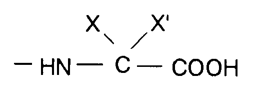 où X est l'hydrogène, un alkyle en C₁-C₅ ou un radical carbocyclique non aromatique en C₃-C₁₅ et X' est l'hydrogène, ou bien X et X', ensemble avec l'atome de carbone auquel ils sont liés, forment un carbocycle non aromatique en C₃-C₁₅ et leurs sels.

**APPLICATION :** antagonistes de la neurotensine

## Description

La présente invention concerne de nouveaux N-oxydes de 1-(7-chloroquinoléin-4-yl)pyrazole-3-carboxamides substitués ayant une grande affinité pour le récepteur de la neurotensine, un procédé pour leur préparation et des compositions pharmaceutiques les contenant en tant que principes actifs.

Les premiers médicaments potentiels de synthèse, non peptidiques, capables de se lier aux récepteurs de la neurotensine ont été décrits dans EP-0477049. Il s'agit d'amides de l'acide pyrazole-3-carboxylique, diversement substitués par des acides aminés, qui déplacent la neurotensine iodée de son récepteur, à des doses inférieures à la micromole, sur des membranes de cerveau humain. Cette série a conduit à la mise au point d'un composé, l'acide 2-{[1-(7-chloroquinoléin-4-yl)-5-(2,6-diméthoxyphényl)pyrazol-3-yl]carbonylamino}adamantane-2-carboxylique, dénommé ci-après SR48692, doté d'une activité antagoniste de la neurotensine puissante et sélective (D. Gully et al., Proc. Natl. Acad. Sci. USA, 1993, *90*, 65-69).

La caractéristique de la série de produits décrite dans EP-0477049 est la présence, en position 1 du cycle pyrazole, notamment d'un groupe phényle, naphtyle, 4-quinoléinyle, substitués ou non substitués. Plus particulièrement, le SR48692 possède, en position 1 du pyrazole, un groupe 7-chloroquinoléin-4-yle.

Il a été maintenant trouvé qu'en oxydant, dans des conditions douces, l'azote du groupe 7-chloroquinoléin-4-yle des dérivés de pyrazole-3-carboxamides, on obtient des molécules qui, présentent, par rapport à leurs précurseurs non N-oxydes, au moins la même activité vis-à-vis des récepteurs de la neurotensine, et qui possèdent de plus une meilleure solubilité, en particulier dans l'eau.

Ainsi, ces composés nouveaux selon l'invention sont particulièrement intéressants car ils permettent la préparation de solutions injectables.

En outre, ils sont compatibles avec de nombreuses formulations galéniques administrables par voie orale car ils possèdent une meilleure biodisponibilité.

La présente invention concerne donc, selon un de ses aspects, des N-oxydes de 1-(7-chloroquinoléin-4-yl)pyrazole-3-carboxamides substitués de formule : 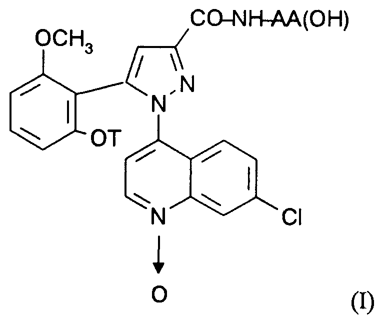
dans laquelle
- T représente l'hydrogène, un alkyle en C₁-C₄, un cycloalkyle en C₃-C₈, un (C₃-C₈) cycloalkylméthyle, un méthoxyéthyle;
- le groupe -NH-AA(OH) représente le résidu d'un acide aminé 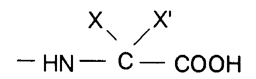 où X est l'hydrogène, un alkyle en C₁-C₅ ou un radical carbocyclique non aromatique en C₃-C₁₅ et X' est l'hydrogène, ou bien X et X', ensemble avec l'atome de carbone auquel ils sont liés, forment un carbocycle non aromatique en C₃-C₁₅ ; et leurs sels.

Par alkyle en C₁-C₄ ou alkyle en C₁-C₅, on entend des alkyles droits ou ramifiés.

Les radicaux carbocycliques non aromatiques en C₃-C₁₅ comprennent les radicaux mono ou polycycliques, condensés ou pontés, saturés ou insaturés, éventuellement terpéniques. Ces radicaux sont éventuellement mono- ou poly-substitués par un alkyle en C₁-C₄.

Les radicaux monocycliques incluent les cycloalkyles en C₃-C₁₂ par exemple cyclopropyle, cyclopentyle, cyclohexyle, cycloheptyle, cyclooctyle, cyclododécyle.

Dans le résidu de l'acide aminé ci-dessus lorsque X et X' ensemble avec l'atome de carbone auquel ils sont liés forment un carbocycle non aromatique en C₃-C₁₅, ledit carbocycle est tel que défini pour les radicaux correspondants ci-dessus.

Parmi les carbocycles non aromatiques polycycliques, l'adamantane est le préféré. Ainsi lorsque X' est l'hydrogène X représente un groupe 1-adamantyle ou 2-adamantyle et lorsque -C(XX') ensemble forment un carbocycle, ce radical représente le radical 2-adamantylidène.

Parmi les carbocycles non aromatiques, le cyclopentane et le cyclohexane sont particulièrement préférés.

Les N-oxydes de quinoléinyl-pyrazole substitués préférés selon la présente invention sont ceux de formule (I) dans laquelle:

T représente un groupe méthyle ou cyclopropylméthyle et le groupe -NH-AA(OH) représente le résidu de l'acide 2-aminoadamantane-2-carboxylique ou (S) 2-amino-2-cyclohexylacétique et leurs sels.

Les sels sont ceux avec des métaux alcalins, de préférence le sodium ou le potassium, des métaux alcalino-terreux, de préférence le calcium et avec des bases organiques comme la diéthylamine, la trométhamine, la méglumine (N-méthyl-D-glucamine), la lysine, l'arginine, l'histidine ou la diéthanolamine.

Les sels des composés de formule I selon la présente invention comprennent également ceux avec des acides minéraux ou organiques qui permettent une séparation ou une cristallisation convenable des composés de formule I, tels que l'acide picrique, l'acide oxalique ou un acide optiquement actif, par exemple un acide mandélique ou un acide camphosulfonique, et ceux qui forment des sels pharmaceutiquement acceptables tels que le chlorhydrate, I'hydrogénosulfate, le dihydrogénophosphate, le méthanesulfonate, le maléate, le fumarate, le 2-naphtalène-2-sulfonate, l'iséthionate.

Lorsque les composés (I) comportent un carbone asymétrique, les énantiomères font partie de l'invention.

Lorsque le groupe -NH(AA)OH représente le résidu d'un aminoacide cycloaliphatique, les composés de formule (I) comprennent aussi bien ceux pour lesquels la fonction amine est en position endo par rapport au système cyclique aliphatique que ceux pour lesquels la fonction amine est en position exo par rapport au système cyclique aliphatique.

Selon un autre de ses aspects, la présente invention concerne un procédé pour la préparation des N-oxydes de 1-(7-chloroquinoléin-4-yl)pyrazole-3-carboxamides substitués de formule (I) et de leurs sels avec des bases minérales ou organiques caractérisé en ce que l'on traite un dérivé de formule : 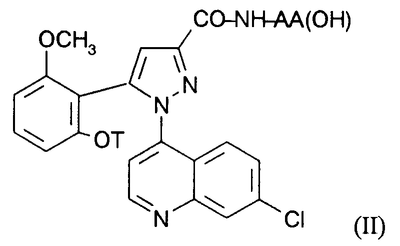
dans laquelle T et AA(OH) ont les significations données ci-dessus pour le composé de formule (I) avec un agent d'oxydation à température ambiante dans un solvant aprotique pour obtenir les composés (I) ou un de leurs sels.

Les agents d'oxydation utilisés sont bien connus de l'homme de l'art et sont par exemple choisis parmi :
. l'hexahydrate de magnésium monoperoxyphtalate (ou HMPP) 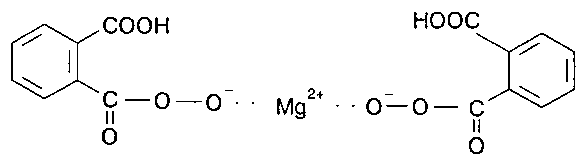
. l'acide perbenzoïque
. l'acide métachloroperbenzoïque (ou mCPBA)
. l'acide perphtalique
. l'acide performique
. l'acide peracétique.

On peut également utiliser d'autres peracides.

Les solvants utilisés sont ceux classiquement utilisés par l'homme du métier pour des réactions d'oxydation, comme par exemple des solvants aprotiques dipolaires, tels que le diméthylformamide ou des solvants chlorés, tel que le dichlorométhane ou le chloroforme.

Un agent oxydant préféré est l'acide métachloroperbenzoïque qui conduit à de bons rendements dans des conditions d'oxydation douces. La température de réaction est de préférence la température ambiante, ce qui permet d'éviter la formation de produits de dégradation ou d'hydroxylation.

Les composés de formule (II) sont préparés selon EP-0477049, selon le SCHEMA 1 suivant:

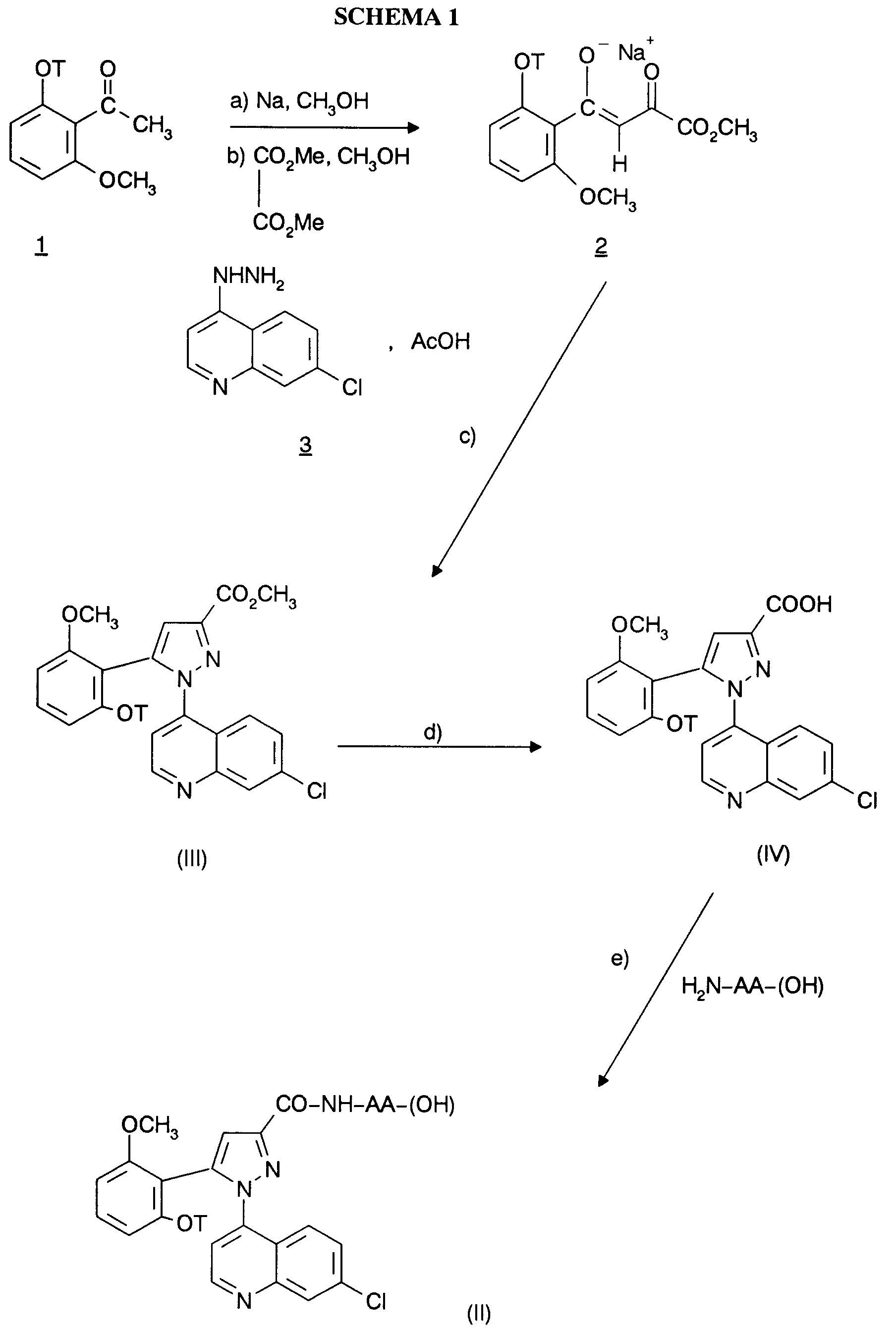

Dans la première étape a) on fait réagir une base forte, tel que le méthylate de sodium sur une cétone de formule 1 dans laquelle T est tel que défini précédemment, puis on fait agir (étape b)) une quantité équimolaire d'oxalate de méthyle dans un alcanol comme par exemple le méthanol, selon L. Claisen, Ber., 1909, *42*, 59. Après précipitation dans un éther, tel que l'éther diéthylique ou l'éther diisopropylique, les énolates de sodium 2 sont séparés par filtration. On peut également préparer un énolate de lithium selon W.V. Murray et al., J. Heterocyclic Chem., 1989, 26, 1389.

L'énolate métallique 2 ainsi préparé et un excès de dérivé de 7-chloro-4-(hydrazin-1-yl)quinoléine 3 ou un de ses sels sont alors chauffés au reflux de l'acide acétique (étape c)) pour obtenir les esters (III).

Par saponification des esters (III) par action d'un agent alcalin comme par exemple l'hydroxyde de potassium ou l'hydroxyde de sodium puis acidification, on obtient les acides (IV) (étape d)).

Comme dérivé fonctionnel de l'acide (7-chloroquinoléin-4-yl)pyrazole-3-carboxylique substitué de formule (IV), on peut utiliser le chlorure d'acide, l'anhydride, un anhydride mixte, un ester alkylique en C₁-C₄, un ester activé, par exemple l'ester de p-nitrophényle, ou l'acide libre opportunément activé, par exemple, avec le N,N-dicyclohexylcarbodiimide ou avec l'hexafluorophosphate de benzotriazol-N-oxytris-(diméthylamino)-phosphonium (BOP).

Les acides aminés de formule NH₂-AA-(OH) peuvent être utilisés soit tels quels, soit après protection préalable par des groupements protecteurs habituels en synthèse peptidique.

Ainsi dans l'étape e) du procédé on peut faire réagir le chlorure d'un acide 1-(7-chloroquinoléin-4-yl)pyrazole-3-carboxylique, obtenu par réaction du chlorure de thionyle sur un acide de formule (IV), avec un acide aminé dans un solvant tel que l'acétonitrile, le THF, le DMF ou le dichlorométhane, sous une atmosphère inerte, à la température ambiante, pendant un temps compris entre quelques heures et quelques jours, en présence d'une base telle que la pyridine, l'hydroxyde de sodium ou la triéthylamine.

Une variante de l'étape e) consiste à préparer le chlorure d'acide ou l'anhydride mixte d'un acide (7-chloroquinoléin-4-yl)pyrazolecarboxylique par réaction du chloroformiate d'isobutyle ou d'éthyle avec un acide de formule (IV), et à le faire réagir avec un dérivé N,O-bis-triméthylsilylé d'un acide aminé obtenu selon une adaptation de la méthode décrite dans la publication de M.T. Nagasawa et al. J. Med. Chem., 1975, *18,8*,826-830, par réaction du bis(triméthylsilyl)acétamide ou du 1,3-bis(triméthylsilyl)urée ou du bis(trifluorométhyl)acétamide avec un acide aminé de formule NH₂-AA-(OH) dans des solvants, tels que l'acétonitrile, le dichlorométhane, sous atmosphère inerte, à la température ambiante, ou au reflux du solvant pendant un temps compris entre quelques heures à un jour.

Une autre variante au mode opératoire de l'étape e) consiste à faire réagir l'anhydride mixte d'un acide pyrazole-3-carboxylique avec un acide aminé de formule NH₂-AA-(OH), dans un solvant, tel que le dichlorométhane, sous une atmosphère inerte, à la température ambiante, pendant un temps compris entre un jour et quelques jours, en présence d'une base telle que la triéthylamine.

Les acides (7-chloroquinoléin-4-yl)pyrazole-3-carboxyliques de formule (IV): 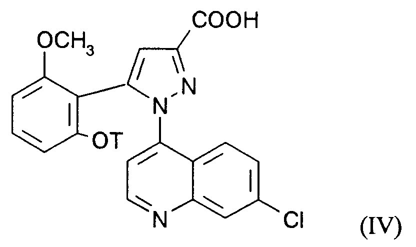
dans laquelle T représente un cycloalkyle en C₃-C₈, un cycloalkylméthyle en C₃-C₈ ou un méthoxyéthyle ainsi que les dérivés fonctionnels de la fonction acide sont nouveaux et, en tant qu'intermédiaires-clés dans la préparation des composés (I) constituent un aspect ultérieur de la présente invention.

Les composés de formule (II): 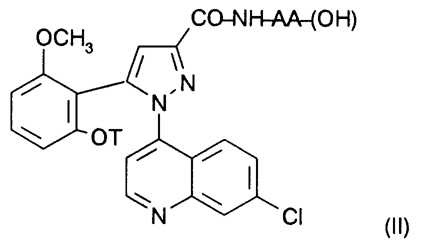
dans lesquels T représente un cycloalkyle en C₃-C₈, un cycloalkylméthyle en C₃-C₈ ou un méthoxyéthyle sont également nouveaux et constituent un autre aspect de l'invention.

Lorsque le produit de formule (II) est obtenu sous forme acide, il peut être transformé en un sel métallique, notamment alcalin, tel que le sel de sodium, ou alcalino-terreux, tel que le sel de calcium, selon les procédés classiques.

Les aminoacides non commerciaux sont préparés selon la synthèse de Strecker, Ann. 1850, *75*, 27 ou selon la synthèse de H.T. Bucherer et al. J. Pract. Chem. 1934, *141*, 5, suivie d'une hydrolyse pour conduire aux aminoacides ; par exemple, l'acide 2-aminoadamantane-2-carboxylique est préparé selon H.T. Nagasawa et al., J. Med., Chem., 1973,*16*, (7), 823 ou selon M. Paventi et al. Can. J. Chem. 1987, *65*, 2114.

Les acides α-amino-1-adamantylacétique et a-amino-2-adamantylacétique sont préparés selon B. Gaspert et al., Croatico Chemica Acta, 1976, *48*, (2), 169-178.

L'acide 2-aminonorbornane-2-carboxylique est préparé selon H.S. Tager et al. J. Am. Chem. Soc., 1972, *94*, 968.

Les acides α-aminocycloalkylcarboxyliques sont préparés selon J.W. Tsang et al., J. Med. Chem., 1984, *27*, 1663.

Les cyclopentylglycines R et S sont préparées selon la demande de brevet européen EP-477049.

Les cyclohexylglycines R et S sont préparées selon Rudman et al., J. Am. Chem. Soc., 1952, *74*, 551.

On peut également préparer les cyclohexylglycines R et S par hydrogénation catalytique des phénylglycines R et S.

On peut aussi préparer les acides α-aminocycloalkylcarboxyliques de configuration R ou S par hydrolyse enzymatique, stéréospécifique, des dérivés N-acétylés racémiques correspondants selon J. Hill et al., J. Org. Chem., 1965, 1321.

Les composés de formule (I) ci-dessus comprennent également ceux dans lesquels un ou plusieurs atomes d'hydrogène ou de carbone ont été remplacés par leur isotope radioactif par exemple le tritium ou le carbone-14. De tels composés marqués sont utiles dans des travaux de recherche, de métabolisme ou de pharmacocinétique, dans des essais biochimiques en tant que ligands de récepteurs.

Les composés de formule (I) et leurs sels avec des bases minérales ou organiques possèdent une très grande affinité pour les récepteurs humains de la neurotensine, dans les tests décrits dans la publication de D. Gully et al. citée ci-dessus. Plus particulièrement, par rapport aux dérivés 1-naphtyl et 4-chloro-1-naphtyl décrits dans EP-0477049, qui ont une CI₅₀ égale ou supérieure à 100 nM, les composés de l'invention possèdent une CI₅₀ nettement inférieure, allant de quelques nM jusqu'à 50 nM. Particulièrement intéressants sont les produits de formule (I) dans laquelle T est méthyle ou cyclopropylméthyle, plus particulièrement l'amide avec l'acide-2-amino-2-adamantanecarboxylique qui ont une CI₅₀ de l'ordre de 2 nM.

Ce composé est donc même plus actif que le SR48692, ce qui est inattendu si on se réfère à l'activité déjà très élevée des composés décrits dans EP-0477049.

De plus, les composés N-oxydes, en particulier celui décrit à l'EXEMPLE 2 ci-après, l'acide 2-{[1-(1-oxyde-7-chloroquinoléin-4-yl)-5-(2,6-diméthoxyphényl) pyrazol-3-yl]carbonylamino}adamantane-2-carboxylique ont fait l'objet d'une étude de solubilité comparée avec le SR48692.

Les milieux étudiés sont: l'eau, l'éthanol et un mélange eau/polyéthylèneglycol 400 (70/30 v/v).

Les mesures de solubilités ont été réalisées à 25°C après agitation pendant 3 ou S heures à saturation.

Après s'être placé dans des conditions selon lesquelles les produits solubilisés dans l'eau ne sont pas adsorbés, sur certains filtres, les solutions sont filtrées puis dosées par chromatographie liquide (Colonne µBondapak C18, éluant acétonitrile acide trifluoroacétique, détection à 254 nm, débit 1 ml/minute).

Les résultats obtenus sont les suivants :

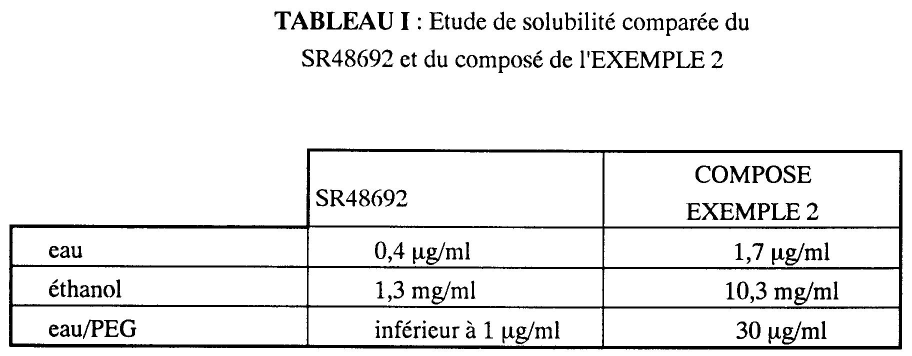

Cette étude montre la solubilité supérieure et inattendue du composé de l'EXEMPLE 2 dans, en particulier, l'eau et le mélange eau/polyéthylèneglycol qui sont des solvants permettant la préparation de formes injectables. A titre d'exemple complémentaire, on a indiqué la solubilité des composés dans l'éthanol.

Les composés de la présente invention sont peu toxiques ; notamment, leur toxicité aiguë est compatible avec leur utilisation comme médicaments. Pour une telle utilisation, on administre aux mammifères une quantité efficace d'un composé de formule (I), ou d'un de ses sels pharmaceutiquement acceptables, pour le traitement d'états pathologiques associés à un dysfonctionnement des systèmes dopaminergiques, par exemple comme antipsychotiques [D.R. Handrich et al., Brain Research, 1982, *231*, 216-221 et C.B. Nemeroff, Biological Psychiatry, 1980, *15*, (2), 283-302], dans les désordres des systèmes cardiovasculaire ou gastrointestinal.

Ainsi la présente invention a pour objet, selon un autre de ses aspects, des compositions pharmaceutiques contenant, comme principes actifs, les composés de formule (I) ou leurs sels éventuels pharmaceutiquement acceptables.

Dans les compositions pharmaceutiques de la présente invention pour l'administration orale, sublinguale, sous-cutanée, intramusculaire, intraveineuse, transdermique ou rectale, les principes actifs peuvent être administrés, sous formes unitaires d'administration, ou en mélange avec des supports pharmaceutiques classiques, aux animaux et aux êtres humains. Les formes unitaires d'administration appropriées comprennent les formes par voie orale, telles que les comprimés, les gélules, les poudres, les granules et les solutions ou suspensions orales, les formes d'administration sublinguale et buccale, les formes d'administration sous-cutanée, intramusculaire ou intraveineuse et les formes d'administration rectale.

Afin d'obtenir l'effet désiré, la dose de principe actif peut varier entre 0,5 et 1 000 mg par jour, de préférence entre 2 et 500 mg.

Chaque dose unitaire peut contenir de 0,5 à 250 mg de principe actif, de préférence de 1 à 125 mg, en combinaison avec un véhicule pharmaceutiquement acceptable. Cette dose unitaire peut être administrée 1 à 4 fois par jour.

Lorsqu'on prépare une composition solide sous forme de comprimés, on mélange le principe actif avec un véhicule pharmaceutiquement acceptable, tel que la gélatine, l'amidon, le lactose, le stéarate de magnésium, le talc, la gomme arabique, ou analogues. On peut enrober les comprimés de saccharose ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélule en mélangeant le principe actif avec un diluant et en versant le mélange obtenu dans des gélules molles ou dures.

Une préparation sous forme de sirop ou d'élixir peut contenir le principe actif conjointement avec un édulcorant, acalorique de préférence, du méthylparaben et du propylparaben comme antiseptique, ainsi qu'un agent donnant du goût et un colorant approprié.

Les poudres ou les granules dispersibles dans l'eau peuvent contenir le principe actif en mélange avec des agents de dispersion, des agents mouillants, ou des agents de mise en suspension, tels que la polyvinylpyrrolidone et similaires, de même qu'avec des édulcorants ou des correcteurs du goût.

Le principe actif peut être également présenté sous forme de complexe avec une cyclodextrine, par exemple α, β, γ dextrine, 2-hydroxypropyl-β-cyclodextrine, méthyl-β-cyclodextrine.

Pour une administration rectale on recourt à des suppositoires qui sont préparés avec des liants fondant à la température rectale, par exemple du beurre de cacao ou des polyéthylèneglycols.

Le principe actif peut être formulé également sous forme de microcapsules, éventuellement avec un ou plusieurs supports.

Les exemples suivants, donnés à titre non limitatif, illustrent l'invention. Dans les PREPARATIONS ci-après, sont décrites les méthodes de synthèse des différents intermédiaires permettant d'obtenir les composés de l'invention.

Les points de fusion ont été mesurés au banc chauffant Koffler.

Les composés selon l'invention possèdent une analyse centésimale conforme à la théorie.

Les spectres de résonnance magnétique nucléaire et les spectres de masse sont également conformes à la structure des composés décrits dans les EXEMPLES ci-après.

### PREPARATION I

5 g de 2-hydroxy-6-méthoxyacétophénone sont mis en solution dans 100 ml d'isopropanol en présence de 1,2 équivalents (6,3 ml) d'hydroxyde de césium à 50 % dans l'eau. Le mélange est agité pendant dix minutes, puis concentré sous vide et repris dans l'isopropanol et concentré sous vide. Le résidu est repris dans 30 ml de diméthylformamide puis on ajoute une solution de 1,2 équivalents (3,5 ml) de bromure de cyclopropylméthyle et chauffe le mélange réactionnel à 80 °C pendant 4 heures. On concentre sous vide, reprend le résidu dans l'acétate d'éthyle, lave successivement avec une solution saturée de NaCl, à l'eau, décante la phase organique, sèche et concentre sous vide pour obtenir 5,1 g de 2-cyclopropylméthyloxy-6-méthoxyacétophénone attendue.

### PREPARATION II

On dissout 0,53 g de sodium dans 15 ml de méthanol puis on ajoute une solution de 5,1 g du composé préparé précédemment et de 1 équivalent (3,4 g) d'oxalate de méthyle dans 25 ml de méthanol. Le mélange réactionnel est chauffé à reflux pendant 6 heures puis refroidi. On ajoute de l'éther isopropylique jusqu'à précipitation et sépare le précipité par filtration pour obtenir 5,3 g du sel de sodium de l'ester méthylique de l'acide 4-(2-cyclopropylméthyloxy-6-méthoxyphényl)-2,4-dioxobutanoïque attendu.

### PREPARATION III

1 g du sel de sodium préparé précédemment et 1,1 équivalent (0,65 g) de 7-chloro-4-(hydrazin-1-yl)quinoléine sont mis en suspension dans 10 ml d'acide acétique. On chauffe le mélange réactionnel à 100°C pendant 5 heures puis on le verse dans 150 ml d'eau glacée et sépare le précipité par filtration pour obtenir 0,74 g de l'ester méthylique de l'acide 5-(2-cyclopropylméthyloxy-6-méthoxyphényl)-1-(7-chloroquinoléin-4-yl)pyrazole-3-carboxylique attendu.

### PREPARATION IV

2,7 g de l'ester préparé précédemment sont mis en solution dans un mélange de 25 ml de méthanol et de 25 ml d'eau en présence de 2,5 équivalents (0,815 g) d'hydroxyde de potassium. Le mélange réactionnel est chauffé à reflux pendant deux heures puis versé dans de l'eau glacée. On extrait à l'éther diéthylique puis on acidifie la phase éthérée avec une solution d'acide chlorhydrique (pH = 2), sépare le précipité par filtration et le rince à l'eau pour obtenir 2,5 g de l'acide 5-(2-cyclopropylméthyloxy-6-méthoxyphényl)-1-(7-chloroquinoléin-4-yl)pyrazole-3-carboxylique attendu.

### PREPARATION V

A 39 g d'acide 2-aminoadamantane-2-carboxylique dans 680 ml d'acétonitrile, on ajoute à température ambiante 2,1 équivalents (42,7 g) de bistriméthylsilylacétamide puis on chauffe le mélange réactionnel à reflux pendant deux heures. La solution devient limpide et contient le N,O-bistriméthylsilyle de l'acide 2-amino-2-adamantane carboxylique.

### PREPARATION VI

1,35 g de l'acide pyrazolecarboxylique obtenu selon la PREPARATION IV sont mis en solution dans 30 ml de toluène en présence de 2,25 ml de chlorure de thionyle. Le mélange réactionnel est chauffé à reflux pendant 5 heures puis concentré sous vide. On ajoute le chlorure d'acide ainsi obtenu à une solution obtenue selon la PREPARATION V et contenant l'équivalent de 0,59 mg de l'acide adamantanecarboxylique. Ce mélange réactionnel est alors chauffé à reflux pendant 3 heures puis on concentre le solvant sous vide. Le résidu est repris dans un mélange de 12 ml de méthanol et de 2 ml d'eau puis agité pendant 1 heure à température ambiante au cours de laquelle le produit attendu précipite. On complète la précipitation en fin d'hydrolyse par l'addition de 10 ml d'eau. On laisse sous agitation pendant une demi heure puis on sépare le précipité par filtration et le lave successivement à l'eau, au pentane puis à l'éther diéthylique pour obtenir, après séchage, 1,8 g de l'acide 2-{[1-(7-chloroquinoléin-4-yl)-5-(2-cyclopropylméthyloxy-6-méthoxyphényl)-pyrazol-3-yl]carbonyl}adamantane-2-carboxylique ; F = 200°C.

### EXEMPLE 1

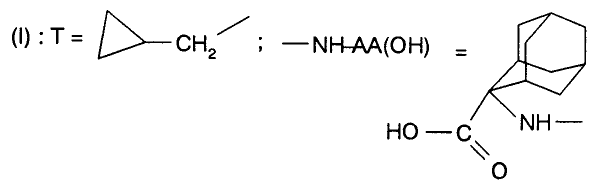

0,626 g de l'acide obtenu à la PREPARATION VI est mis en solution dans 100 ml de chloroforme en présence de 0,294 mg d'acide métachloroperbenzoïque et le mélange est laissé sous agitation pendant 24 heures à température ambiante. Le mélange réactionnel est concentré sous vide puis repris dans 10 ml de dichlorométhane. Le précipité obtenu est séparé par filtration puis lavé au dichlorométhane pour fournir 0,24 g de 2-{[1-(1-oxyde-7-chloroquinoléin-4-yl)-5-(2-cyclo-propylméthyl-oxy-6-méthoxyphényl)-pyrazol-3-yl]carbonylamino}adamantane-2-carboxylique; F = 190°C.

### EXEMPLE 2

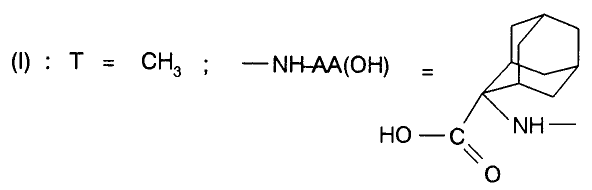

On prépare l'acide 2-{[1-(7-chloroquinoléin-4-yl)-5-(2,6-diméthoxyphényl)pyrazol-3-yl]carbonylamino}adamantane-2-carboxylique (SR 48692) en procédant selon les modes opératoires décrits dans les préparations I à VI ci-dessus en utilisant du bromure méthyle à la place du bromure de cyclopropylméthyle. 0,5 g de cet acide est mis en solution dans 80 ml de diméthylformamide puis on ajoute 4,15 g de hexahydrate de magnésium monopéroxyphtalate. Le mélange réactionnel est placé sous agitation et abandonné à température ambiante pendant 24 heures. On ajoute alors 250 ml d'une solution d'acide trifluoroacétique à 1‰ dans l'eau et on extrait successivement avec 150 ml puis 100 ml puis 50 ml de dichlorométhane. Les fractions d'extraction sont rassemblées et lavées deux fois avec 250 ml d'eau distillée. L'extrait obtenu après lavage est concentré sous vide à 40°C, le résidu est ensuite purifié par HPLC préparative sur phase de silice. Pour cela, il est repris dans 4,5 ml d'éluant constitué par le mélange dichlorométhane/isopropanol 97/3 (v/v) et élué sur une phase Kromasil 100Å-10µ sous 40 bars. Les fractions sont collectées toutes les 0,2 minute soit un volume de 25 ml par fraction. La concentration des fractions de produit pur fournit 0,080 g de cristaux blancs qui sont lavés au dichlorométhane pour conduire finalement à 0,050 g d'acide 2-{[1-(1-oxyde-7-chloroquinoléin-4-yl)-5-(2,6-diméthoxyphényl)-3-pyrazolyl]carbonylamino}adamantane-2-carboxylique ; F = 205°C.

## Revendications

1. Composé de formule 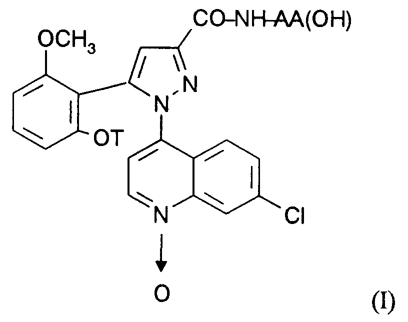 dans laquelle
- T représente l'hydrogène, un alkyle en C₁-C₄, un cycloalkyle en C₃-C₈, un (C₃-C₈) cycloalkylméthyle, un méthoxyéthyle;
- le groupe -NH-AA(OH) représente le résidu d'un acide aminé de formule 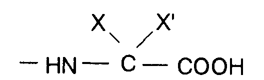 où X est l'hydrogène, un alkyle en C₁-C₅ ou un radical carbocyclique non aromatique en C₃-C₁₅ et X' est l'hydrogène, ou bien X et X', ensemble avec l'atome de carbone auquel ils sont liés, forment un carbocycle non aromatique en C₃-C₁₅ et ses sels.

2. Composé selon la revendication 1 dans lequel T représente un groupe méthyle ou cyclopropylméthyle et le groupe -NH-AA(OH) représente le résidu de l'acide 2-aminoadamantane-2-carboxylique et ses sels.

3. Procédé pour la préparation des composés de formule (I) selon la revendication 1 et de leurs sels, caractérisé en ce que l'on traite un dérivé de formule : 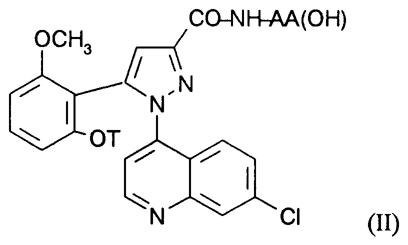 dans laquelle T et -NH-AA(OH) ont les significations données dans la revendication 1 pour le composé de formule (I) avec un agent d'oxydation à température ambiante dans un solvant aprotique pour obtenir les composés (I) ou un de leurs sels.

4. Acide de formule : 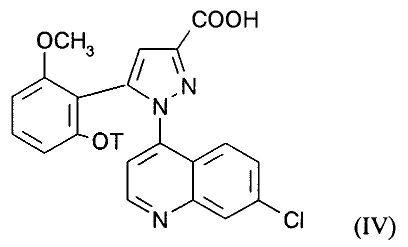 dans lequel T représente un cycloalkyle en C₃-C₈, un cycloalkylméthyle en C₃-C₈ ou un méthoxyéthyle.

5. Amide de formule : 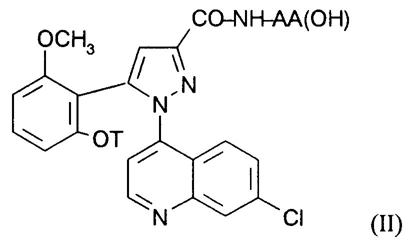 dans lequel T représente un cycloalkyle en C₃-C₈, un cycloalkylméthyle en C₃-C₈ ou un méthoxyéthyle et NH-AA(OH) est tel que défini dans la revendication 1.

6. Composition pharmaceutique contenant en tant que principe actif un composé selon l'une quelconque des revendications 1 à 2 ou un des ses sels éventuels pharmaceutiquement acceptables.

7. Composition pharmaceutique selon la revendication 6 sous forme d'unité de dosage.

8. Composition pharmaceutique selon la revendication 7, caractérisée en ce qu'elle contient de 0,5 à 250 mg de principe actif en mélange avec au moins un véhicule pharmaceutiquement acceptable.
